# EUROPEAN PATENT APPLICATION

(11) **EP 1 832 593 A1**
(43) Date of publication of application: **12.09.2007**
(21) Application number: 06119163.1
(22) Date of filing: 18.08.2006
(51) Int. Cl.: C07D 501/00

(54) **Direct process for the production of sterile Cefepime dihydrochloride monohydrate**

(30) Priority: 09.03.2006 IT MI20060422
(71) Applicant: Harvest Lodge Limited, London, WC2A 3LJ (GB)
(72) Inventor: Zenoni, Maurizio, 20067 Paullo (MI) (IT); Filippi, Mauro, 20060 Bettolino di Mediglia (MI) (IT)
(74) Representative: Frignoli, Luigi

(57) **Abstract**

An amino acid in solution is precipitated with concentrated hydrochloric acid and isolated as the dihydrochloride monohydrate. Said dihydrochloride is redissolved and reprecipitated by adding a solvent.

## Description

The present invention relates to a process for preparing sterile Cefepime dihydrochloride monohydrate.

US Patent 4910301 (column 11) and its related patents (e.g. US 4994451) describe the preparation of cefepime dihydrochloride monohydrate from cefepime sulphate. The process comprises precipitating the sulphate as a means of purifying the cefepime obtained in the synthesis, its subsequent transformation into the zwitterion and its passage from this to cefepime dihydrochloride monohydrate by acidification with HCl and dilution with acetone until precipitation of the dihydrochloride monohydrate is complete.

It has now been surprisingly discovered that the aforedescribed process can be simplified by avoiding precipitation of the cefepime sulphate derived from the synthesis and instead precipitating the cefepime dihydrochloride monohydrate directly. In this respect, the aqueous solution containing the cefepime derived from the synthesis is decolorized with carbon, filtered, washed with water and methanol, then acidified with concentrated HCl to crystallize the aforesaid dihydrochloride by diluting with acetone. The dihydrochloride thus obtained is dissolved in methanol or water, filtered sterilely and added dropwise to acetone. The sterile product is obtained by filtering the suspension containing acetone and methanol or acetone and water.

Specifically, the process of the invention is characterised in that a solution of cefepime obtained from the synthesis is decolorized with carbon, treated with concentrated HCl to pH 0.4-0.6 at a temperature between 15° and 30°C, then allowed to crystallize for 15-60 minutes and subsequently diluted by adding a water miscible organic solvent over 60-90 minutes at 20°-30°C until complete precipitation of the crude cefepime dihydrochloride monohydrate, which is then filtered off, redissolved in a solvent chosen from the group consisting of methanol and water at 15°-25°C, filtered sterilely, diluted with the same organic solvent used previously over 30-60 minutes, in order to induce crystallization, and finally diluted again with the same solvent over 90-150 minutes to complete crystallization of the sterile cefepime dihydrochloride monohydrate, which is filtered off, washed with acetone and dried under vacuum to a K.F. between 3.0% and 4.5%. It is therefore evident that the process of the present invention provides some considerable advantages, such as an appreciable reduction in working hours, no sodium sulphate to dispose of, absence of ash in the final product because sulphuric acid is not used.

It was also observed that by using very pure materials for the synthesis together with very careful and attentive monitoring of the process, a final synthesis aqueous solution can be obtained which is so pure as to enable cefepime dihydrochloride monohydrate to be obtained of such purity that a simple sterile filtration of the final synthesis aqueous solution enables sterile cefepime dihydrochloride monohydrate to be precipitated, thus avoiding the second step of purification and sterilization, with an immense yield advantage of a 10% increase, which is added to the already indicated advantages for the process in the two aforedescribed steps.

A further and unexpected advantage is the fact that the sterile cefepime dihydrochloride monohydrate prepared in accordance with the process of the present invention, presents a density almost double that of sterile cefepime dihydrochloride monohydrate obtained by known methods. This fact represents an undoubted advantage because filtration and washing are facilitated, as is its dispensing into sterile containers, with the sterile product occupying less space in the warehouse and during transport, before its distribution into the sterile containers used in clinical practice.

The process outlined above will now be described in detail with the examples that follow:

### EXAMPLE 1

### Crude cefepime dihydrochloride monohydrate

290 of a solution of rich liquors derived from the synthesis and containing about 65 g of cefepime as internal salt, are decolorized with 1.5 g of carbon while agitating for 20 minutes at ambient temperature. The mixture is filtered and washed with 43 ml of water and 10 ml of methanol. Agitation is maintained between 25° and 30°C while concentrated HCl (91.5 g) is added dropwise. The mixture is then seeded and allowed to crystallize for 30 minutes. Completion of the crystallization is achieved by adding acetone (3.3 I) dropwise over 60 minutes at 25°C. The product is filtered off, washed with acetone and dried at 40°C under vacuum. Yield: 74 g of crude cefepime dihydrochloride monohydrate, equal to 90% of the theoretical on the starting nucleus, with 84.7% purity.

### EXAMPLE 2

### Sterile cefepime dihydrochloride monohydrate

20 g of crude cefepime dihydrochloride monohydrate are dissolved in methanol (85 ml) at ambient temperature. The solution obtained is filtered sterilely then maintained between 18° and 22°C under agitation while acetone (50 ml) is added dropwise over 45 minutes. The mixture is seeded and allowed to crystallize for 2 hours; further acetone (450 ml) is added over 2 hours, then the product is filtered off, washed with acetone and dried at 45°C under vacuum to a K.F. between 3.0% and 4.5%.

Yield: 18.6 g of sterile cefepime dihydrochloride monohydrate, equal to 93% of the theoretical relative to the crude product. The density of the product obtained is 0.55 g/ml, while under the same conditions the density of a sample prepared inn accordance with the known art is less than 0.3 g/ml.

Superimposable results can be obtained by dissolving the crude cefepime dihydrochloride monohydrate in water instead of methanol and using a final synthesis aqueous solution obtained from very pure raw materials, then by conducting the synthesis with scrupulous care the sterile cefepime dihydrochloride monohydrate is obtained with yields of 90% on the starting nucleus.

The yields obtained by operating in accordance with the known method are 90% of cefepime sulphate on the original nucleus, whereas, with the transformation of cefepime sulphate into sterile cefepime dihydrochloride monohydrate, a yield of 90% is obtained: it is therefore evident that although in the first step of the process there is exact equivalence between the known art and the process of the present invention, in the second and final step a clear increase in the yield (3%) is obtained if operating in accordance with the present invention. Differences between the two products at the analytical level have not been found other than the different densities of the crystals and the absence of ash in the product obtained in accordance with the process of the present invention.

## Claims

1. A process for producing sterile cefepime dihydrochloride monohydrate, **characterised in that** a cefepime solution obtained from the synthesis is decolorized with carbon, treated with concentrated HCl to pH 0.4-0.6 at a temperature between 15° and 30°C, then allowed to crystallize for 15-60 minutes and subsequently diluted by adding a water miscible organic solvent over 60-90 minutes at 20°-30°C until complete precipitation of the crude cefepime dihydrochloride monohydrate, which is then filtered off, redissolved in a solvent chosen from the group consisting of methanol and water at 15°-25°C, filtered sterilely, diluted with the same already used organic solvent over 30-60 minutes in order to induce crystallization, and finally again diluted with the same solvent over 90-150 minutes to complete crystallization of the sterile cefepime dihydrochloride monohydrate, which is filtered off, washed with acetone and dried under vacuum to a K.F. between 3.0% and 4.5%.

2. A process for producing sterile cefepime dihydrochloride monohydrate, **characterised in that** a particularly pure aqueous solution of cefepime obtained from the synthesis is decolorized with carbon, filtered sterilely, treated with concentrated HCl to pH 0.4-0.6 at a temperature between 15° and 30°C, then allowed to crystallize for 15-60 minutes and subsequently diluted by adding a water miscible organic solvent over 60-90 minutes at 20°-30°C until complete precipitation of the sterile cefepime dihydrochloride monohydrate, which is then filtered off, washed with acetone and dried under vacuum to a K.F. between 3.0% and 4.5%.

3. A process as claimed in claims 1 and 2, **characterised in that** said acidification with concentrated HCl is undertaken until pH 0.5 is achieved.

4. A process as claimed in claims 1 to 3, **characterised in that** said organic solvent is acetone.

5. A process as claimed in claims 1, 3 and 4, **characterised in that** the crude cefepime dihydrochloride monohydrate is dissolved in methanol, to be then filtered sterilely.

6. A process as claimed in claims 1, 3 and 4, **characterised in that** the crude cefepime dihydrochloride monohydrate is dissolved in water, to be then filtered sterilely.

7. A process as claimed in claims 1 to 6, **characterised in that** the sterile cefepime dihydrochloride monohydrate obtained has a density about double that of the product prepared by the known method.
